# EUROPEAN PATENT APPLICATION

(11) **EP 4 249 599 A1**
(43) Date of publication of application: **27.09.2023**
(21) Application number: 21894040.1
(22) Date of filing: 19.11.2021
(51) Int. Cl.: C12N 15/867, C12N 5/10, A61K 35/17, A61P 35/02

(54) **PREPARATION METHOD AND APPLICATION OF CD7-CAR-T CELLS**

(30) Priority: 23.11.2020 CN 202011325596
(71) Applicant: Persongen Biotherapeutics (Suzhou) Co., Ltd., Suzhou, Jiangsu 215000 (CN); Persongen.Anke Cellular Therapeutics Co., Ltd., Hefei, Anhui 230088 (CN)
(72) Inventor: YANG, Lin, Jiangsu 215000 (CN); WANG, Min, Hefei, Anhui 230088 (CN); WANG, Yu, Hefei, Anhui 230088 (CN); PAN, Guifang, Hefei, Anhui 230088 (CN)
(74) Representative: dompatent von Kreisler Selting Werner - Partnerschaft von Patent- und Rechtsanwälten mbB
(86) International application number: PCT/CN2021/131917
(87) International publication number: WO 2022/105893

(57) **Abstract**

Provided are a preparation method and an application of CD7-CAR-T cells. The method comprises: (i) providing a sample to be processed containing T cells, (ii) sorting and activating the T cells contained in the sample, so as to obtain activated T cells, (iii) introducing a first viral vector for expressing a CD7 blocking molecule into the activated T cells, so as to obtain CD7 blocked T cells, and (iv) introducing a second viral vector for expressing a CD7-CAR into the CD7-blocked T cells to obtain CD7-CAR-T cells. By adjusting the transfection sequence and transfection time of the lentivirus expressing the CD7 blocking molecule and the lentivirus expressing the CD7-CAR, transfection efficiency is improved, and the cytotoxicity of the CAR-T cells is enhanced.

## Description

### Technical field

The present invention is related to the field of biotechnology, and more particularly to the preparation method and use of a CD7-CAR-T cell.

### Background

T-cell derived malignancies represent a typical type of blood system cancer, with quite high recurrence and mortality rates in both pediatric and adult patients. There is currently no highly effective therapy. T-cell acute lymphoblastic leukemia (T-ALL) is a highly heterogeneous hematological malignancy, accounting for approximately 25% of adult acute lymphoblastic leukemia cases and approximately 15% of pediatric acute lymphoblastic leukemia cases. Clinical symptoms of T-ALL mainly include infection, anemia, fever, abnormal bleeding and the like. T-ALL progresses rapidly and can infiltrate into lymph nodes, liver, spleen, central nervous system and testicles and other tissues and organs in a short period. Patients may die soon after the onset of the disease in case of no promptly and effective treatment. Currently, treatment strategies of T-cell acute lymphoblastic leukemia (T-ALL) mainly include intensive chemotherapy, allogeneic hematopoietic stem cell transplantation (allo-HSCT), antiviral therapy and molecular targeted therapy. However, intensive chemotherapy usually can not prevent disease recurrences after treatment. For those patients who relapse after the first treatment, the remission rate of salvage chemotherapy is only about 20-40%. In addition, although hematopoietic stem cell transplantation may be the only curable option at present, it may also be associated with a life-threatening risk caused by graft-versus-host diseases.

In recent years, chimeric antigen receptor T cell (CAR-T) therapy has shown very significant clinical therapeutic effects as a new adoptive immunotherapy technique for various solid tumors and hematological tumors, among which the treatment effect of therapy for B-cell derived lymphocytic leukemia and lymphoma is most significant. CAR-T therapy genetically modifies T lymphocytes of patients, in order to target and eliminate malignant tumors in a major histocompatibility complex-independent manner. One of the key factors to the effective application of this technology is to select a suitable target to construct corresponding CAR molecules. The best target antigen should be expressed only in tumor cells, not in normal cells, or may on certain cells that have a clinical response plan after the normal cells are temporarily killed.

CD7 molecules are highly expressed in most T-cell blood tumor cells. CD7 is a transmembrane glycoprotein expressed by T and NK cells and their precursor cells, which binds to its ligand K12/SECTM1 and subsequently stimulates T cell activation. However, it seems that CD7 molecules do not play a key role in the development or function of T cells, since disruption of CD7 molecules in progenitor cells of murine T cells does not affect normal T cell development and homeostasis, and does not affect the efficacy of T cells. Importantly, CD7 has been used as a target of antibody for treating T-cell lymphoma, and patients do not experience serious adverse effects after CD7-targeting therapy. Therefore, CD7 molecules may be a very suitable therapeutic target for T-cell blood tumors. However, the targeting treatment of T cell acute lymphoblastic leukemia with CD7-CAR-T cells still faces great problems since both normal effector T cells and T cell tumors express CD7 antigen, which leads to the fratricide of CD7-CAR-T cells, and it is difficult to prepare CD7-CAR-T cells successfully *in vitro.*

Therefore, there is an urgent need in this field to develop a new preparation method for CD7-CAR-T cells.

### Summary of the invention

The purpose of the invention is to provide a preparation method and use of CD7-CAR-T cell.

Particularly, the present invention provides a preparation method of CD7-CAR-T cells based on humanized CD7 nanobody.

The present invention further provides the *in vitro* killing effect of CD7-CAR T cells constructed by using PA007CD and PA107CD vectors on CD7-positive tumor cells.

In the first aspect of the present invention, it provides a method for preparing CD7-CAR-T cells, which comprises the following steps:
(i) providing a sample to be processed containing T cells,
(ii) sorting and activating the T cells contained in the sample, thereby obtaining activated T cells,
(iii) introducing a first viral vector for expressing a CD7-blocking molecule into the activated T cells, thereby obtaining CD7-blocked T cells, and
(iv) introducing a second viral vector for expressing a CD7-CAR into the CD7-blocked T cells, thereby obtaining the CD7-CAR-T cells,
wherein, after the T cells are co-incubated with an activator for 12-36 h, preferably 18-30 h, more preferably 22-26 h, the first viral vector for expressing a CD7-blocking molecule is introduced into the activated T cells.

In another preferred embodiment, interval between the introduction of the first viral vector and the introduction of the second viral vector is 36-60 h, preferably 42-54 h, and more preferably 46-50 h.

In another preferred embodiment, the following steps are comprised in step (iii) :
(a) co-culturing the first viral vector for expressing the CD7-blocking molecule and the activated T cells under a condition suitable for transfection for 36-60 h, preferably 42-54 h, and more preferably 46-50 h, thereby obtaining a first transfection mixture;
(b) removing the first viral vector from the first transfection mixture, thereby obtaining the CD7-blocked T cells.

In another preferred embodiment, step (b) further comprises a step of removing the activator added in step (ii).

In another preferred embodiment, in step (b), the first viral vector and the activator in the first transfection mixture are removed.

In another preferred embodiment, the following steps are comprised in step (iv) :
(c) co-culturing the second viral vector for expressing the CD7-CAR and the CD7-blocked T cells under a condition suitable for transfection for 12-36 h, preferably 18-30 h, and more preferably 22-26 h, thereby obtaining a second transfection mixture;
(d) removing the second viral vector from the second transfection mixture, thereby obtaining the CD7-CAR-T cells.

In another preferred embodiment, the first viral vector and the second viral vector are lentiviral vectors.

In another preferred embodiment, the co-culture is performed at 36-38 °C.

In another preferred embodiment, in step (a), the amount ratio of the first viral vectors to T cells (multiplicity of infection) is 1:0.05-0.4.

In another preferred embodiment, in step (c), the amount ratio of the second viral vectors to T cells (multiplicity of infection) is 1:0.5-4.

In another preferred embodiment, the T cells are washed with buffer solution to remove the viral vector.

In another preferred embodiment, the sample is peripheral blood mononuclear cells.

In another preferred embodiment, in step (ii), the sorting and activation are performed by using an activator.

In another preferred embodiment, the activator is magnetic beads targeting CD3 and CD28.

In another preferred embodiment, the magnetic beads are Miltenyi Transact which has the characteristics of easy removal, degradability and good activation effect. Miltenyi Transact is the magnetic beads targeting CD3 and CD28 used in Examples.

In another preferred embodiment, the ratio of T cells to activator is 1×10⁶ cells : 5-20 ul.

In another preferred embodiment, the magnetic beads targeting CD3 and CD28 are co-incubated with the T cells for 12-36 h, preferably 18-30 h, and more preferably 22-26 h, followed by introduction of the first viral vector for expressing a CD7-blocking molecule into the activated T cells.

In another preferred embodiment, the following step is further comprised after step (iv):
(v) the CD7-CAR-T cells are expanded under a condition suitable for T cell expansion.

In another preferred embodiment, the antigen-binding domain of the CD7-CAR comprises one or more anti-CD7 nanobodies.

In another preferred embodiment, the amino acid sequence of the CD7 nanobody is shown in SEQ ID NO: 13.

In another preferred embodiment, the CAR further comprises a transmembrane region derived from ICOS and/or an intracellular co-stimulatory region derived from ICOS.

In another preferred embodiment, the CAR comprises an intracellular co-stimulatory region derived from ICOS and an intracellular co-stimulatory region derived from 4-1BB.

In another preferred embodiment, the structure of the CAR is shown in the following Formula I:

L-VHH-H-TM-C-CD3ζ (I)

wherein,
each "-" is independently a linking peptide or peptide bond;
L is a signal peptide sequence;
VHH is an antigen binding domain comprising one or two anti-CD7 nanobodies;
H is a hinge region;
TM is a transmembrane domain;
C is a co-stimulatory signaling molecule;
CD3ζ is a cytoplasmic signal transduction sequence derived from CD3ζ.

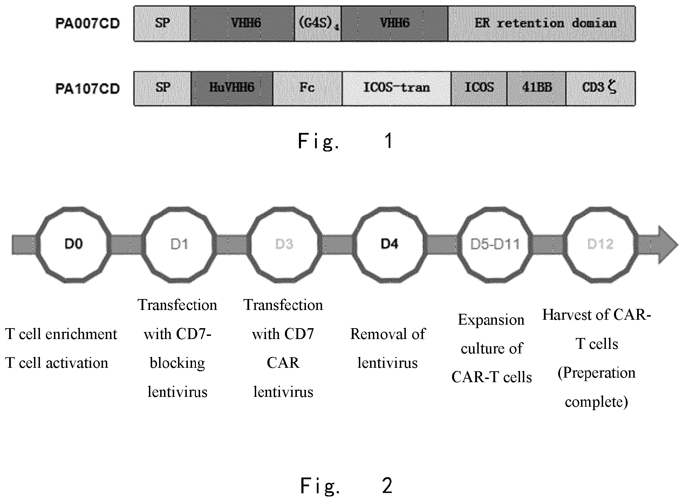

In another preferred embodiment, the structure of the CAR is shown in Fig. 1.

In another preferred embodiment, L is a signal peptide of a protein selected from the group consisting of CD8, CD28, GM-CSF, CD4, CD137 and a combination thereof.

In another preferred embodiment, L is a signal peptide derived from CD8.

In another preferred embodiment, the amino acid sequence of L is shown in SEQ ID NO: 4.

In another preferred embodiment, the VHH comprises two anti-CD7 nanobodies, preferably the two anti-CD7 nanobodies are linked with a linking peptide, and more preferably the linking peptide is shown in SEQ ID NO: 8.

In another preferred embodiment, the nucleotide sequence of the linking peptide is shown in SEQ ID NO: 7.

In another preferred embodiment, the anti-CD7 nanobody comprises a humanized nanobody and a camel nanobody, and preferably is a humanized nanobody.

In another preferred embodiment, the VHH is one humanized anti-CD7 nanobody.

In another preferred embodiment, H is a hinge region derived from a protein selected from the group consisting of Fc, CD8, CD28, CD137 and a combination thereof.

In another preferred embodiment, H is an Fc fragment, and preferably the Fc fragment is a human IgG4 Fc fragment.

In another preferred embodiment, the amino acid sequence of the Fc fragment is shown in SEQ ID NO: 14.

In another preferred embodiment, TM is a transmembrane region of a protein selected from the group consisting of ICOS, CD28, CD3 epsilon, CD45, CD4, CD5, CD8, CD9, CD16, CD22, CD33, CD37, CD64, CD80, CD86, CD134, CD137, CD 154 and a combination thereof.

In another preferred embodiment, TM is a transmembrane region derived from ICOS.

In another preferred embodiment, the amino acid sequence of the transmembrane region derived from ICOS is shown in SEQ ID NO: 15.

In another preferred embodiment, C is a co-stimulatory signaling molecule of a protein selected from the group consisting of ICOS, OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2 and a combination thereof.

In another preferred embodiment, C is a co-stimulatory signaling molecule derived from ICOS and/or 4-1BB.

In another preferred embodiment, the amino acid sequence of the co-stimulatory signaling molecule derived from ICOS is shown in SEQ ID NO: 16.

In another preferred embodiment, the amino acid sequence of the co-stimulatory signaling molecule derived from 4-1BB is shown in SEQ ID NO: 17.

In another preferred embodiment, the amino acid sequence of CD3ζ is shown in SEQ ID NO: 18.

In another preferred embodiment, the CAR further comprises a cell suicide element (preferably at C-terminus).

In another preferred embodiment, the CAR is linked with the cell suicide element via a self-cleaving element.

In another preferred embodiment, the cell suicide element is linked with CD3ζ of the CAR via a T2A.

In another preferred embodiment, the cell suicide element is selected from the group consisting of HSV-TK, iCasp9, ΔCD20, mTMPK, ΔCD19, RQR8, EGFRt and a combination thereof.

In another preferred embodiment, the cell suicide element is tEGFR.

In another preferred embodiment, the amino acid sequence of the tEGFR is shown in SEQ ID NO: 20.

In another preferred embodiment, the amino acid sequence of T2A is shown in SEQ ID NO: 19.

In another preferred embodiment, the CAR is PA107CD CAR (i.e., The CD7 CAR named PA3-17 in Chinese Patent Application No. 201911150678.7).

In another preferred embodiment, the amino acid sequence of the CAR is shown in SEQ ID NO: 12.

In another preferred embodiment, the CD7-blocking molecule comprises one or more anti-CD7 nanobodies and an endoplasmic reticulum retention sequence.

In another preferred embodiment, the CD7-blocking molecule has a structure as shown in the following Formula II:

L'-VHH'-ER (II)

wherein,
each "-" is independently a linking peptide or peptide bond;
L' is a signal peptide sequence;
VHH' is a binding region comprising two anti-CD7 nanobodies; and
ER is an endoplasmic reticulum retention sequence.

In another preferred embodiment, the amino acid sequence of L' is shown in SEQ ID NO: 4.

In another preferred embodiment, the nucleotide sequence of L' is shown in SEQ ID NO: 3.

In another preferred embodiment, the two anti-CD7 nanobodies of the VHH' are linked with a linking peptide, and more preferably the linking peptide is shown in SEQ ID NO: 8.

In another preferred embodiment, the amino acid sequence of the VHH' is shown in SEQ ID NO: 6.

In another preferred embodiment, the nucleotide sequence of VHH' is shown in SEQ ID NO: 5.

In another preferred embodiment, the VHH' is a camel nanobody.

In another preferred embodiment, the amino acid sequence of the ER is shown in SEQ ID NO: 10.

In another preferred embodiment, the nucleotide sequence of ER is shown in SEQ ID NO: 9.

In another preferred embodiment, the amino acid sequence of the CD7-blocking molecule is shown in SEQ ID NO: 2.

In another preferred embodiment, the nucleotide sequence of the CD7-blocking molecule is shown in SEQ ID NO: 1.

In the second aspect of the present invention, it provides a CD7-CAR-T cell prepared by the method according to the first aspect of the present invention.

In another preferred embodiment, at least 80%, preferably 90%, more preferably 95%, and most preferably 98% of endogenous CD7 expression is blocked in the CD7-CAR-T cell.

In the third aspect of the present invention, it provides a formulation comprising the CD7-CAR-T cell according to the second aspect of the present invention, and a pharmaceutically acceptable carrier.

In another preferred embodiment, the preparation is a liquid formulation.

In another preferred embodiment, the formulation is an injection.

In another preferred embodiment, the concentration of the engineered immune cell in the formulation is 1×10³-1×10⁸ cells/ml, and preferably 1×10⁴-1×10⁷ cells/ml..

The present invention also provides a method of treating a disease, which comprises administering an appropriate amount of the CD7-CAR-T cell according to the second aspect of the present invention or the formulation according to the third aspect of the present invention, to a subject in need of treatment.

In another preferred embodiment, the disease is cancer or tumor.

It should be understood that, within the scope of the present invention, the technical features specifically described above and below (such as the Examples) can be combined with each other, thereby constituting a new or preferred technical solution which needs not be described one by one.

### Description of Figure

Figure 1 shows vector structure diagrams of the CD7-CAR and CD blocking molecule of the present invention.
Figure 2 shows a preparation flowchart of the CD7-CAR T cell of the present invention.
Figure 3 shows the flow cytometry of CD7 molecule expression on the surface of T cells (CD7 molecule expression was detected by using CD7 antibodies in the FITC channels) and transfection efficiency of CD7-CAR T cells (T cell expression was detected by using CD3 antibodies in the PE channel and CAR expression was detected by using FC antibodies in the APC channel).
Figure 4 shows the cytotoxicity of CD7-CAR-T cells to 293T cells overexpressing CD7.
Figure 5 shows the cytotoxicity of CD7-CAR-T cells to acute lymphoid leukemia cells (CEM) naturally expressing CD7.
Figure 6 shows cytokine secretion of CD7-CAR-T cells after incubation with 293T-CD7 cells.
Figure 7 shows cytokine secretion of CD7-CAR-T cells after incubation with CEM cells.

### EMBODIMENTS

After extensive and intensive research, the inventors discovered a preparation method and use of a CD7-CAR-T cell. Specifically, by adjusting the transfection order and transfection time of the lentivirus expressing the CD7-blocking molecule and the lentivirus expressing the CD7-CAR, the method of the present invention improves the transfection efficiency, and enhances the cytotoxicity of the CAR-T cells. On this basis, the present invention has been completed.

Specifically, the present invention aims to provide a method for preparing CD7-CAR-T cells based on a humanized CD7 nanobody sequence. The preparation of CD7-CAR-T cells involves the transfection of two kinds of lentiviruses, one is PA007CD virus that anchors the T cell surface-expressed CD7 antigen to endoplasmic reticulum or golgi apparatus matrix of the cell, and the other is PA107CD lentivirus capable of expressing CD7-CAR on the surface of T cells. The inventor discovered that, factors such as the order of use of the two lentiviruses and the time interval between transfections can directly affect the quality of the final CAR-T cells. The present invention describes a method for preparing CD7-CAR-T cells comprising transfecting PA007CD lentivirus 24 hours after T cell sorting and activation, washing to remove PA007CD lentivirus and activator after 48 hours later, and then transfecting PA107CD lentivirus for 24 hours followed by another wash to remove PA107CD lentivirus. The CD7-CAR-T cells prepared by this method shows high CAR transfection efficiency and good cell viability. *In vitro* functional experiments have shown that the CD7-CAR-T cell prepared in this application is able to specifically kill CD7-positive target cells and secrete high level of specific cytokines.

### Terms

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the present invention into a subject using any one of various methods and delivery systems known to those skilled in the art, including intravenous, intramuscular, subcutaneous, intraperitoneal, spinal or other parenteral administration, such as by injection or infusion.

The term "antibody" (Ab) may include, but is not limited to, an immunoglobulin that specifically binds an antigen and contains at least two heavy (H) chains and two light (L) chains linked by disulfide bonds, or an antigen binding parts thereof. Each H chain contains a heavy chain variable region (abbreviated herein as VH) and a heavy chain constant region. The heavy chain constant region contains three constant domains, CH1, CH2, and CH3. Each light chain contains a light chain variable region (abbreviated herein as VL) and a light chain constant region. The light chain constant region contains a constant domain CL. The VH and VL regions can be further subdivided into hypervariable regions called complementarity determining regions (CDR), which are interspersed within more conservative regions called framework regions (FR). Each VH and VL contains three CDRs and four FRs, which are arranged from amino terminal to carboxy terminal in the following order: FR1, CDR1, FR2, CDR2, FR3, CDR3, FR4. The variable regions of the heavy and light chains contain a binding domain that interacts with an antigen.

It should be understood that the names of amino acids herein are represented by the internationally accepted single English letter, to which the corresponding three English letter names of amino acid are as follows: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y) and Val (V).

Sequecnes of the CD7 blocking molecule (PA007CD), CD7-CAR (PA107CD) and elements thereof are provided in the sequence listing of the present application. Meanings represented by each sequence are shown in following Table 1.

**Table.1 Names of sequences in the sequence list**

| Sequence | Name |
|---|---|
| SEQ ID NO: 1 | Base sequence of CD7 blocking molecule |
| SEQ ID NO: 2 | Amino acid sequence of CD7 blocking molecule |
| SEQ ID NO: 3 | Base sequence of SP(signal peptide) |
| SEQ ID NO: 4 | Amino acid sequence of SP(signal peptide) |
| SEQ ID NO: 5 | Base sequence of CD7 nanobody (VHH6) |
| SEQ ID NO: 6 | Amino acid sequence of CD7 nanobody (VHH6) |
| SEQ ID NO: 7 | Base sequence of (G4S)4 |
| SEQ ID NO: 8 | Amino acid sequence of (G4S)4 |
| SEQ ID NO: 9 | Base sequence of ER |
| SEQ ID NO: 10 | Amino acid sequence of ER |
| SEQ ID NO: 11 | Base sequence of PA107CD |
| SEQ ID NO: 12 | Amino acid sequence of PA107CD |
| SEQ ID NO: 13 | Amino acid sequence of HuVHH6 (humanized sequence of CD7 nanobody) |
| SEQ ID NO: 14 | Amino acid sequence of Fc (human IgG4 Fc sequence, hinge region) |
| SEQ ID NO: 15 | Amino acid sequence of ICOS-tran (ICOS transmembrane region sequence) |
| SEQ ID NO: 16 | Amino acid sequence of ICOS (ICOS intracellular region sequence) |
| SEQ ID NO: 17 | Amino acid sequence of 4-1BB (4-1BB intracellular region sequence) |
| SEQ ID NO: 18 | Amino acid sequence of CD3ζ |
| SEQ ID NO: 19 | Amino acid sequence of T2A |
| SEQ ID NO: 20 | Amino acid sequence of tEGFR (truncated EGFR) |

### Chimeric antigen receptor (CAR)

The design of CARs has gone through the following process. The first generation CAR has only one intracellular signal component, CD3ζ or FcyRI molecule. Because there is only one activation domain in the cell, it can only cause transient T cell proliferation and less cytokine secretion, and does not provide long-term T cell proliferation signals and sustained antitumor effects *in vivo.* Therefore, it has not achieved very good clinical efficacy. In the second generation CAR, based on the original structure, a co-stimulatory molecule such as CD28, 4-1BB, OX40 and ICOS is introduced. Compared with the first generation CAR, the function has been greatly improved, and the sustainability of CAR-T cells and the ability to kill tumor cells are further enhanced. Based on the second generation CAR, some new immune stimulatory molecules such as CD27 and CD134 are linked in tandem to develop the third and fourth generation CARs.

The chimeric antigen receptor (CAR) of the present invention comprises an extracellular domain, a transmembrane domain, and an intracellular domain. The extracellular domain comprises a target-specific binding element (also known as an antigen binding domain). The intracellular domain includes a co-stimulatory signaling region and a ζ chain. The co-stimulatory signaling region refers to a part of the intracellular domain that includes a co-stimulatory molecule. The co-stimulatory molecule is a cell surface molecule required for efficient response of lymphocytes to antigens, rather than a antigen receptor or its ligand.

A linker (or linking peptide) can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

In a preferred embodiment of the present invention, the extracellular domain of the CAR provided by the present invention comprises an antigen binding domain targeting CD7. When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains of the co-stimulatory molecule and the ζ chain. Preferably, the antigen binding domain is fused with an intracellular domain of a combination of an ICOS and/or 4-1BB signaling domain and a CD3ζ signaling domain.

As used herein, the "antigen binding domain" refers to a Fab fragment, a Fab' fragment, a F (ab')2 fragment, or a single Fv fragment that has antigen-binding activity. As a preferred embodiment of the present invention, the antigen binding domain contains one or more nanobodies which specifically recognize CD7.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, transmembrane domains may be selected or modified by amino acid substitutions to avoid binding such domains to the transmembrane domain of the same or different surface membrane proteins, thereby minimizing the interaction with other members of the receptor complexes.

The intracellular domain in the CAR of the present invention comprises the signaling domain of ICOS and/or 4-1BB and the signaling domain of CD3ζ.

The present invention also provides CAR-T cells targeting CD7. Unless otherwise specified, the expression of CD7 on the surface of CAR-T cells of the invention is blocked, preferably by the CD7-blocking molecule described in the second aspect of the invention.

### Suicide gene switch

In order to further control the adverse effects of CAR-T cells such as non-tumor targeting and cytokine release syndrome, the CAR cells of the invention all contain suicide gene switches, which can effectively remove CAR-T cells in the body under the action of exogenous drugs, and block unknown or uncontrollable long-term toxicity, so as to ensure the safety of patients.

The suicide gene switch used in the invention may be HSV-TK, iCasp9, CD20, mTMPK, etc. In comparison, HSV TK, iCasp9 and CD20 have the same ability to remove T cells, but iCasp9 and CD20 remove faster and HSV-TK removes slower.

iCasp9 suicide gene contains a FKBP12-F36V domain, which can be connected via a flexible connector with cysteine aspartate protease 9, which does not contain recruitment domain. The FKBP12-F36V contains a FKBP domain in which phenylalanine replaces valine at the 36th amino acid residue. It has high selectivity and subnanomolar affinity, and can bind with synthetic dimerization ligands, such as AP1903 or other inert small molecules. When small molecules are added, dimerization is promoted and therefore cell apoptosis is induced, while there is ineffective for normal cells without suicide genes.

### Vector

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically.

The present invention also provides vectors in which the expression cassette of the present invention is inserted. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief, the expression cassette or nucleic acid sequence of the present invention is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present invention may also be used for nucleic acid immune and gene therapy, using standard gene delivery protocols. Methods for gene delivery are known in the art. See, e.g., U.S, Pat. Nos. 5,399,346, 5,580,859, and 5,589,466, which are incorporated by reference herein in entirety. In another embodiment, the present invention provides a gene therapy vector.

The nucleic acid can be cloned into various vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al, (2001 , Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for transferring a gene into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters. Inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro*, *ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

### Formulation

The present invention provides a formulation (or preparation) comprising the CAR-T cell according to the first aspect of the present invention, and a pharmaceutically acceptable carrier, diluent or excipient. In one embodiment, the formulation is a liquid preparation. Preferably, the formulation is an injection. Preferably, the concentration of the CAR-T cells in the formulation is 1×103-1×108 cells/ml, more preferably 1×10⁴-1×10⁷ cells/ml.

In one embodiment, the formulation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The formulation of the present invention is preferably formulated for intravenous administration.

### Therapeutic application

The present invention comprises therapeutic applications by using cells (e.g., T cells) transduced with a lentiviral vector (LV) encoding the expression cassette of the present invention. The transduced T cells can target the tumor cell marker CD7, synergistically activate T cells, and cause T cell immune responses, thereby significantly increasing the killing efficiency against tumor cells.

Therefore, the present invention also provides a method for stimulating a T cell-mediated immune response to a target cell population or tissue in a mammal comprising a step of administering to the mammal a CAR-T cell of the present invention.

In one embodiment, the present invention comprises a cell therapy, wherein T cells from autologous patient (or heterologous donor) are isolated, activated and genetically modified to generate CAR-T cells, and then injected into the same patient. The probability of graft-versus-host-disease in the way is extremely low, and antigens are recognized by T cells in a non-MHC-restricted manner. In addition, one CAR-T can treat all cancers that express the antigen. Unlike antibody therapy, CAR-T cells are able to replicate *in vivo* and result in long-term persistence that can lead to sustained tumor control.

In one embodiment, the CAR-T cells of the present invention can undergo robust *in vivo* T cell expansion and can persist for an extended period. In addition, the CAR mediated immune response may be part of an adoptive immunotherapy approach in which CAR-modified T cells induce an immune response specific to the antigen binding moiety in the CAR. For example, an anti-CD7 CAR-T cell elicits an immune response specific against cells expressing CD7.

Although the data disclosed herein specifically disclose lentiviral vector comprising anti-CD7 nanobody, Fc fragment, transmembrane domain, and ICOS intracellular region, 4-1BB and CD3ζ signaling domains, it is contemplated that the present invention include any number of variations for each of the components of the construct as described elsewhere herein.

Cancers that may be treated include tumors that are unvascularized or largely unvascularized, and tumors that are vascularized. Cancers may include non-solid tumors (such as hematological tumors, for example, leukemias and lymphomas) or solid tumors. Types of cancers to be treated with the CARs of the present invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer and ovarian cancer.

The CAR-modified T cells of the present invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cells into a mammal: i) expanding the cells, ii) introducing a nucleic acid encoding a CAR to the cells, and/or iii) cryopreservation of the cells.

Ex vivo procedures are well known in the art and are discussed more fully below. Briefly, cells are isolated from a mammal (preferably a human) and genetically modified (i.e., transduced or transfected *in vitro*) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present invention also provides compositions and methods for *in vivo* immunization to elicit an immune response directed against an antigen in a patient.

The present invention provides a method for treating tumors comprising administering to a subject in need thereof, a therapeutically effective amount of the CAR-modified T cells of the present invention.

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (i.v.) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatments, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the present invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present invention are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to practices in the field. In general, 1×10⁶ to 1×10¹⁰ of the modified T cells of the present invention (e.g., CAR-T20 cells) can be applied to patients by means of, for example, intravenous infusion in each treatment or each course of treatment.

### The main advantages of the present invention include:

(a) CD7-CAR-T cells prepared by the method of the present invention shows high CAR transfection efficiency and good cell viability.
(b) CD7-CAR-T cells prepared by the method of the present invention are able to specifically kill CD7-positive target cells and secrete high level of specific cytokines.

The present invention will be further explained below in conjunction with specific embodiments. It should be understood that these embodiments are only used to illustrate the present invention and not to limit the scope of the present invention. For the experimental methods in the following examples of which the specific conditions are not specifically indicated, they are performed under routine conditions or as instructed by the manufacturers. Unless otherwise specified, percentages and parts are percentages by weight and parts by weight.

### Example 1

### Preparation of CD7-CAR and CD7-blocking molecule

CD7-blocking molecule, whose structure was shown in Fig.1 and amino acid sequence was shown in SEQ ID NO: 2, was constructed by using conventional technical means in the art. Said CD7-blocking molecule was inserted into the lentiviral vectors. Then the CD7-blocking lentiviruse, i.e. PA007CD, was prepared through lentiviral packaging technique.

CD7-CAR whose structure was shown in Fig.1 and amino acid sequence was shown in SEQ ID NO: 12 was constructed by using conventional technical means in the art. Said CD7-CAR was inserted into the lentiviral vector. Then the CD7-CAR lentiviruse, i.e. PA107CD, was prepared through lentiviral packaging technique.

### Example 2

### Preparation of CD7-CAR-T cells

Since more than 90% of human normal T cells express CD7 antigen, it is necessary to block the T cell surface CD7 antigen inside the cell in the process of preparing CD7-CAR-T cells, otherwise there will be fratricide caused by CD7-CAR-T recognization of autologous antigen CD7.

CD7-CAR-T cells were transfected with CD7-blocking lentivirus (PA007CD) so that the T cell-expressed CD7 are blocked in the endoplasmic reticulum or Golgi apparatus and can not be presented to the T cell surface. Then T cells were transfected with CD7-CAR lentivirus (PA107CD), to prepare CD7-CAR-T cells.

In this example, single blood samples from donors were used to obtain mononuclear cells (PBMCs) using Ficoll density gradient centrifugation method, and T cells were enriched from the mononuclear cells by using CD4/CD8 double magnetic beads. T cells, after obtained, were activated with an activator (Transact). On the second day after activation (24 hours after activation), an appropriate number of PA007CD virus were selected for T cell transfection based on the previous validation results, particularly at a ratio of MOI (multiplicity of infection) = 0.05-0.4. At such MOI, CD7 molecules on the surface of T cells can be completely blocked and have little impact on the later PA107CD virus transfection. After 48 hours of PA007CD lentivirus transfection, residual activator and PA007CD lentiviruse was removed by centrifugation, and PA107CD lentiviruse was then added for transfection. The appropriate virus number for PA107CD transfection was also selected based on the previous validation resultsd, particularly at a ratio of MOI (multiplicity of infection) = 0.5- 4. Such MOI ensures transfection efficiency. After 24 hours of transfection, residual PA107CD lentiviruse was removed by centrifugation and the CAR-T cells began to be cultured and expanded. The preparation flowchart is shown in Fig. 2.

### Example 3

### CD7 molecule expression on surface of T cell, and transfection efficiency of CD7-CAR-T

During the preparation process of CD7-CAR-T cells, the expression rate of CD7 on the surface of T cells and the proportion of CAR positive cells (transfection efficiency) are important indicators for evaluating CD7-CAR-T cells. After transfection with PA007CD virus, it is necessary to block the CD7 molecules expression on the surface of T cells, and then CD7-CAR-T cells can be successfully prepared by transfection with PA107CD. 3 batches of CD7-CAR-T cells were prepared by this method. The expression of CD7 molecules and transfection efficiency at the time of sample collection are shown in Table 2.

**Table 2 Efficiency of expression of CD7 molecules on cell surface and transfection**

| **Cell Batch Number** | **LZ012019122101** | **ZL012019123001** | **ZZY12020010701** |
|---|---|---|---|
| CD7 Molecule Expression | 0.20 | 1.05 | 0.99 |
| Transfection Efficiency | 37.80 | 18.86 | 11.56 |

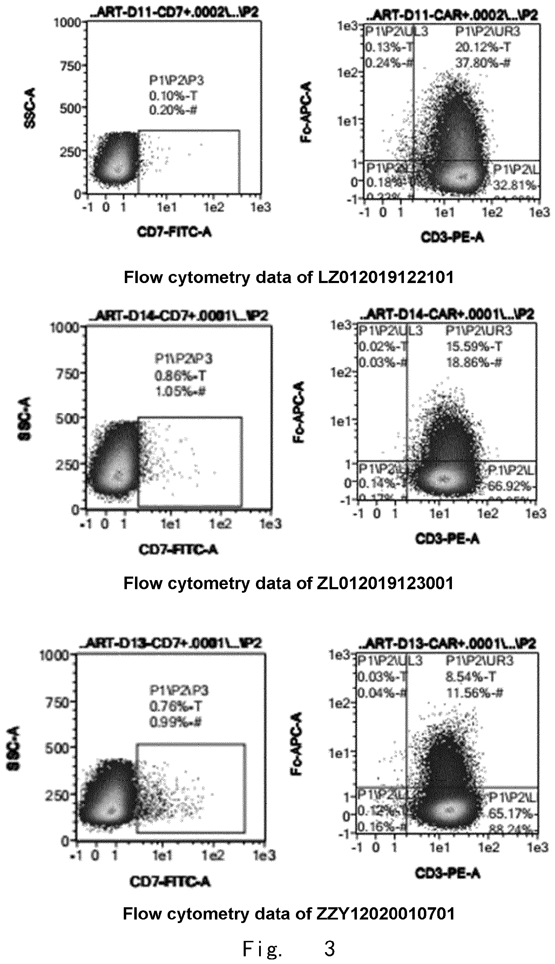

The flow cytometry is shown in Fig. 3.

The results shows that CD7 molecules on the surface of T cells were almost completely blocked after transfection with PA007CD virus, of which the expression amount was about 1%. The transfection of PA107CD lentivirus at this time to prepare CD7-CAR-T reduces the fratricide of cells, which could effectively ensure the viability of CD7 CAR-T cells and significantly improve the success rate of cell preparation.

### Example 4

### Cytotoxicity of CD7-CAR-T cells to 293T cells overexpressing CD7

An important way to determine the biological efficacy of CD7-CAR-T cells is to detect the killing ability of CAR-T cells on target cells after co-incubation of CD7-CAR-T cells with CD7-positive target cells.

293T cells overexpressing CD7 (293T-CD7) were constructed as target cells for evaluation of the *in vitro* killing function of CD7-CAR-T cells. The three groups of CD7-CAR-T cell prepared in Example 2, LZ012019122101, ZL012019123001, and ZZY12020010701, were used for killing experiments at effect-target ratios of 20:1, 10:1 and 5:1, respectively. 293T cells are CD7-negative target cells.

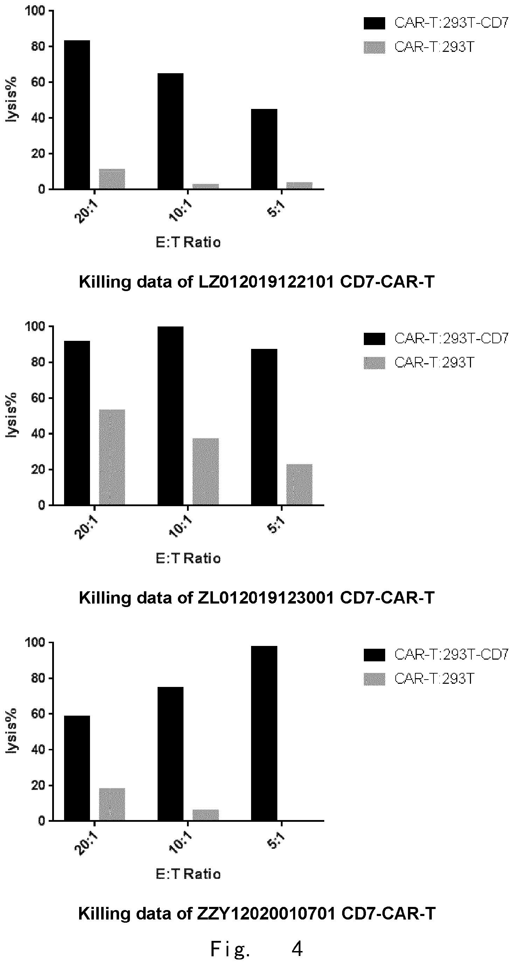

The results are shown in Fig. 4. Through the following killing results on 3 groups of cells, it can be seen that CD7-CAR-T cells have a significant specific killing effect on 293T-CD7 cells (CD7-positive), but no specific killing on 293T cells (CD7-negative), indicating that CD7-CAR-T cells prepared in Example 2 can produce specific cytotoxicity to 293T-CD7 cells overexpressing CD7 positive *in vitro,* but no specific cytotoxicity on CD7-negative 293T cells.

### Example 5

### Cytotoxicity of CD7-CAR-T cells to acute lymphoid leukemia cells (CEM) naturally expressing CD7

CD7-CAR-T cells prepared in Example 2 were used for *in vitro* killing experiments on acute lymphoblastic leukemia cells (CEM) at effect-target ratios of 1:1, 1:10, 1:50 and 1:100, respectively. T cells were used as control effector cells.

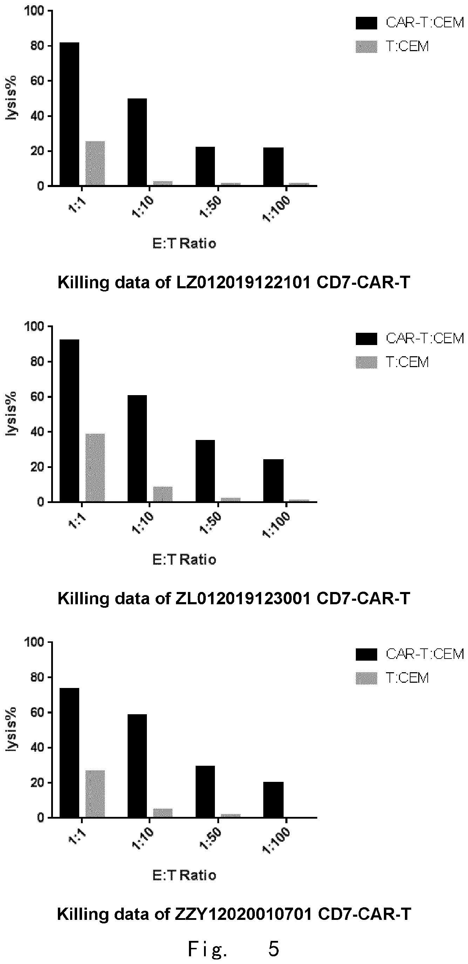

As shown in Fig. 5, compared with the control T cells, CD7-CAR T cells showed significant specific killing effects on CEM, indicating that the CD7-CAR T cells prepared in Example 2 exhibited significant cytotoxicity to CD7-positive CEM cells *in vitro.*

### Example 6

### Cytokine secretion of CD7-CAR-T cells after incubation with 293T-CD7 cells

The cytokine release of CD7-CAR-T cells after incubation with 293T-CD7 cells was analyzed. Supernatant after killing from the group in which effector cells and target cells were co-incubated at an effect-target ratio of 1:1 was selected for detection of the cytokine secretion of IL-2, IFN-γ, and Granzyme B.

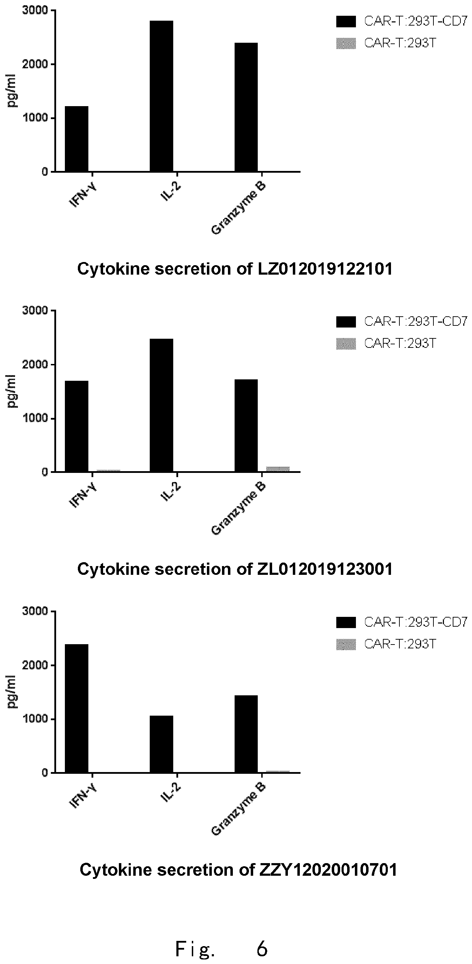

The results are shown in Fig. 6. There is almost no cytokine produced after co-incubation of CD7-CAR-T cells with 293T, while the cytokine release significantly increases after incubation of CD7-CAR-T cells with 293T-CD7 cells. It is indicated that the prepared CD7-CAR-T cells can be specifically activated by CD7-positive 293T-CD7 cells.

### Example 7

### Cytokine secretion of CD7-CAR-T cells after incubation with CEM cells

The cytokine release of CD7-CAR-T cells after incubation with CEM cells was further analyzed. The CD7-CAR-T cells and target cells were incubated at effect-target ratios of 1:10, 1:50, and 1:100, respectively, and the T cell group was used as the control group for detection of the cytokine secretion of IL-2, IFN-γ, and Granzyme B.

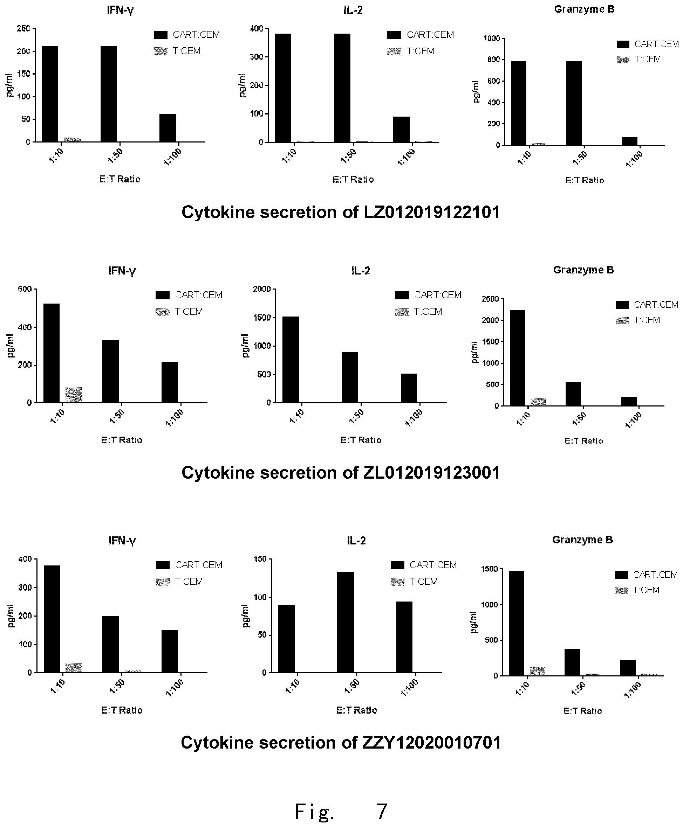

The results are shown in Fig. 7. There is almost no cytokine produced after co-incubation of T cells with target cells, while the cytokine release significantly increases after incubation of CD7-CAR-T cells with CEM cells, which further demonstrates that the prepared CD7-CAR-T cells can be specifically activated by CD7-positive cells.

**Summary:** The main content of the present invention is the successful preparation of CD7-CAR-T cells with high transfection efficiency and good cell activity by a new transfection method, and it is demonstrated that there is a significant specific killing of the prepared CD7-CAR-T cells on CD7-positive tumor cells through a series of *in vitro* functional experiments.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A method for preparing CD7-CAR-T cells, which comprises the following steps:
(i) providing a sample to be processed containing T cells,
(ii) sorting and activating the T cells contained in the sample, thereby obtaining activated T cells,
(iii) introducing a first viral vector for expressing a CD7-blocking molecule into the activated T cells, thereby obtaining CD7-blocked T cells, and
(iv) introducing a second viral vector for expressing a CD7-CAR into the CD7-blocked T cells, thereby obtaining the CD7-CAR-T cells,
wherein, after the T cells are co-incubated with an activator for 12-36 h, preferably 18-30 h, more preferably 22-26 h, the first viral vector for expressing a CD7-blocking molecule is introduced into the activated T cells.

2. The method according to claim 1, wherein the step (iii) comprises the following steps:
(a) co-culturing the first viral vector for expressing the CD7-blocking molecule and the activated T cells under a condition suitable for transfection for 36-60 h, preferably 42-54 h, and more preferably 46-50 h, thereby obtaining a first transfection mixture;
(b) removing the first viral vector from the first transfection mixture, thereby obtaining the CD7-blocked T cells.

3. The method according to claim 2, wherein the step (b) further comprises a step of removing the activator added in step (ii).

4. The method according to claim 1, wherein the step (iv) comprises the following steps:
(c) co-culturing the second viral vector for expressing the CD7-CAR and the CD7-blocked T cells under a condition suitable for transfection for 12-36 h, preferably 18-30 h, and more preferably 22-26 h, thereby obtaining a second transfection mixture;
(d) removing the second viral vector from the second transfection mixture, thereby obtaining the CD7-CAR-T cells.

5. The method according to claim 2, wherein in step (a), the amount ratio of the first viral vectors to T cells (multiplicity of infection) is 1:0.05-0.4.

6. The method according to claim 4, wherein in step (c), the amount ratio of the second viral vectors to T cells (multiplicity of infection) is 1:0.5-4.

7. The method according to claim 1, wherein the following step is further comprised after step (iv):
(v) the CD7-CAR-T cells are expanded under a condition suitable for T cell expansion.

8. The method according to claim 1, wherein the CD7-blocking molecule comprises one or more anti-CD7 nanobodies and an endoplasmic reticulum retention sequence.

9. A CD7-CAR-T cell prepared by the method according to claim 1.

10. A formulation comprising the CD7-CAR-T cell according to claim 9, and a pharmaceutically acceptable carrier.
